# EUROPEAN PATENT APPLICATION

(11) **EP 2 644 102 A1**
(43) Date of publication of application: **02.10.2013**
(21) Application number: 13158331.2
(22) Date of filing: 08.03.2013
(51) Int. Cl.: A61B 8/08, A61B 8/00, G01S 7/52, G06T 5/50

(54) **Method and apparatus for indicating medical equipment on ultrasound image**

(30) Priority: 29.03.2012 KR 20120032581
(71) Applicant: Samsung Medison Co., Ltd., Gangwon-do 250-870 (KR)
(72) Inventor: Kim, Jong-sik, Hongcheon-gun, Gangwon-do (KR); Baek, Ji-hye, Hongcheon-gun, Gangwon-do (KR)
(74) Representative: Schmid, Wolfgang

(57) **Abstract**

A method and apparatus for indicating medical equipment on an ultrasound image, the method including transmitting ultrasound signals toward a target object, receiving ultrasound echo signals at a plurality of angles from the target object and equipment inserted in the target object, obtaining location information of the equipment based on the ultrasound echo signals received at the plurality of angles, generating an image of the equipment based on the obtained location information, generating a basic image based on the ultrasound echo signals received at a predetermined angle, synthesizing the image of the equipment and the basic image, and displaying the synthesized image.

## Description

### CROSS-REFERENCE TO RELATED PATENT APPLICATION

This application claims the benefit of Korean Patent Application No. 10-2012-0032581, filed on March 29, 2012, in the Korean Intellectual Property Office, the disclosure of which is incorporated herein in its entirety by reference.

### BACKGROUND OF THE INVENTION

### 1. Field of the Invention

The present invention relates to a method and apparatus for indicating medical equipment on an ultrasound image, and more particularly, to a method and apparatus for indicating a location of medical equipment on an ultrasound image.

### 2. Description of the Related Art

An ultrasound diagnostic system transmits an ultrasound signal toward a predetermined interior part of a body from a surface of the body of a target object, and obtains an image related to a section of soft tissue or a blood vessel by using information of an ultrasound signal that is passed through tissue inside of the target object or reflected from the tissue inside of the target object.

An ultrasound system has advantages in terms of small size, low cost, and real-time display. Also, the ultrasound system has high stability without exposing patients and users to X-ray radiation, and thus, the ultrasound system is widely used along with other diagnostic imaging systems such as X-ray diagnosis equipment, a computerized tomography (CT) scanner, magnetic resonance imaging (MRI) equipment, nuclear medicine diagnosis equipment, and the like.

Recently, as medical technologies have been developed, a technique is used to perform treatments or examinations by inserting a medical needle for ablation or biopsy in a region of interest while watching an image of the inside of a target object by forming a minimum size hole in the target object without a complete incision. In other words, biopsy needles or syringes are used to perform biopsy, operation, or treatment on a target object. In this case, in order to make a precise diagnosis by using ultrasound diagnostic equipment, indicating a real time location of the biopsy needles or syringes on an ultrasound image is required.

### SUMMARY OF THE INVENTION

The present invention provides a method of indicating medical equipment on an ultrasound image.

The present invention also provides an apparatus for indicating a location of medical equipment on an ultrasound image.

According to an aspect of the present invention, there is provided a method of indicating medical equipment on an ultrasound image. The method may include transmitting ultrasound signals toward a target object, receiving ultrasound echo signals at a plurality of angles from the target object and equipment inserted in the target object, obtaining location information of the equipment based on the ultrasound echo signals received at the plurality of angles, and generating an image of the equipment based on the obtained location information. Also, the method may further include generating a basic image based on the ultrasound echo signals received at a predetermined angle, synthesizing the image of the equipment and the basic image, and displaying the synthesized image.

According to another aspect of the present invention, there is provided an apparatus for indicating a location of medical equipment on an ultrasound image. The apparatus may include a transmitting unit for transmitting ultrasound signals toward a target object, a receiving unit for receiving ultrasound echo signals at a plurality of angles from the target object and equipment that is inserted in the target object, and an equipment information processing unit for obtaining location information of the equipment based on the ultrasound echo signals received at the plurality of angles and generating an image of the equipment based on the obtained location information of the equipment. Also, the apparatus may further include a basic image processing unit for generating a basic image based on the ultrasound echo signal received at a predetermined angle, an image synthesizing unit for synthesizing the image of the equipment and the basic image, and a displaying unit for displaying the synthesized image.

Meanwhile, according to another aspect of the present invention, there is provided a computer readable recording medium on which a program for implementing the method described above.

### BRIEF DESCRIPTION OF THE DRAWINGS

The above and other features and advantages of the present invention will become more apparent by describing in detail exemplary embodiments thereof with reference to the attached drawings in which:
FIG. 1 is a flowchart illustrating an apparatus for indicating medical equipment on an ultrasound image according to an embodiment of the present invention;
FIG. 2 illustrates receiving ultrasound echo signals at a plurality of angles according to an embodiment of the present invention;
FIG. 3A illustrates ultrasound echo signals according to a plurality of angles at which the ultrasound echo signals are received according to an embodiment of the present invention;
FIG. 3B illustrates a height of amplitude of an ultrasound echo signal per each of receiving angles at which the ultrasound echo signals are received;
FIG. 4 illustrates obtaining of location information of equipment by using image information of a plurality of 2D images, which are obtained based on an ultrasound echo signal according to an embodiment of the present invention;
FIG. 5 illustrates an apparatus for indicating medical equipment on an ultrasound image according to an embodiment of the present invention; and
FIG. 6 illustrates an equipment information processing unit according to an embodiment of the present invention.

### DETAILED DESCRIPTION OF THE INVENTION

The terms used in the present application will be briefly described, and then the present invention will be described in detail.

The terms used in the present invention are selected from among common terms that are currently widely used in consideration of their function in the present invention. However, the terms may be different according to an intention of one of ordinary skill in the art, a precedent, or the advent of new technology. Also, in particular cases, the terms are discretionally selected by the applicant of the present invention, and the meaning of those terms will be described in detail in the corresponding part of the detailed description. Therefore, the terms used in the present invention are not merely designations of the terms, but the terms are defined based on the meaning of the terms and content throughout the present invention.

Throughout the present application, when a part "includes" an element, it is to be understood that the part additionally includes other elements rather than excluding other elements as long as there is no particular opposing recitation. Also, the terms such as "... unit", "module", or the like used in the present application indicate an unit, which processes at least one function or motion, and the unit may be implemented by hardware or software, or by a combination of hardware and software.

Throughout the present application, the term "ultrasound image" indicates an image of a target object which is obtained by using ultrasound signals. The term "target object" may indicate a part of a human body. For example, the target object may include a liver, a heart, a uterus, a brain, a breast, organs in abdomen, or a fetus.

Ultrasound images may be implemented in various ways. For example, an ultrasound image may be at least one of a brightness mode (B mode), a color mode (C mode), and a Doppler mode (D mode). Also, according to an embodiment of the present invention, the ultrasound image may be a 2-dimensional (2D) image or a 3-dimensional (3D) image.

Throughout the present application, the term "user" indicates a medical professional such as a doctor, a nurse, a medical technologist, or a medical imaging technologist, but is not limited thereto.

Throughout the present application, the term "medical equipment" may include equipment of an arbitrary shape which is used for performing an operation, biopsy, or intervention on a target object. For example, a medical needle may include a biopsy needle and an ablator needle.

The present invention will now be described more fully with reference to the accompanying drawings for those of ordinary skill in the art to be able to perform the present invention without any difficulty. The invention may, however, be embodied in many different forms and should not be construed as being limited to the embodiments set forth herein; rather, these embodiments are provided so that this disclosure will be thorough and complete, and will fully convey the concept of the invention to those of ordinary skill in the art. Also, parts in the drawings unrelated to the detailed description are omitted to ensure clarity of the present invention. Like reference numerals in the drawings denote like elements, and thus their description will not be repeated.

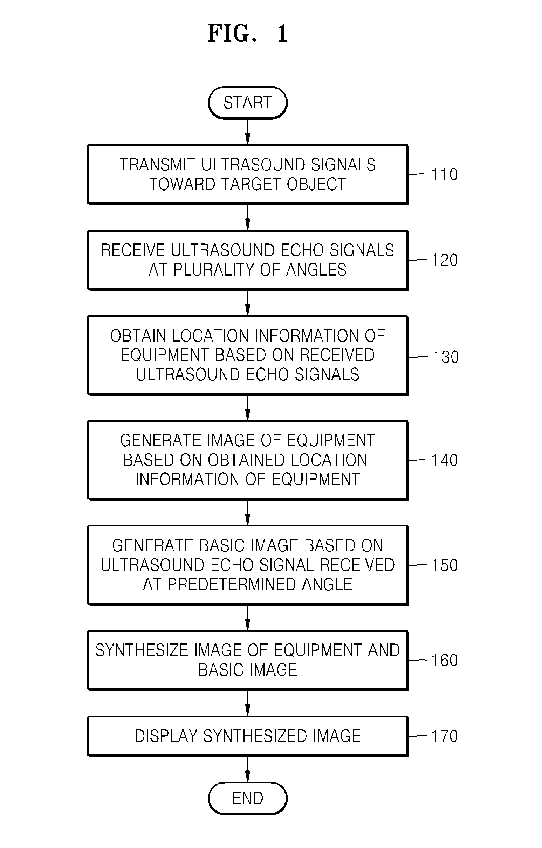

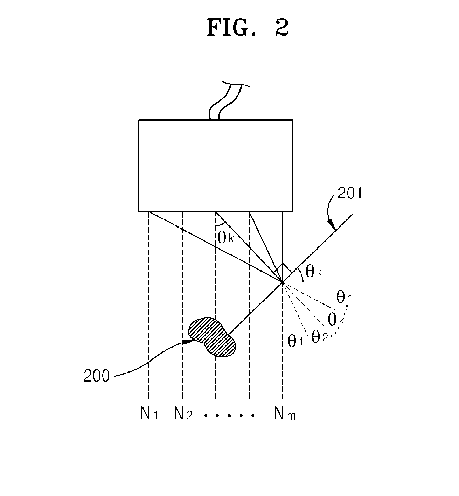

FIG. 1 is a flowchart illustrating an apparatus for indicating medical equipment on an ultrasound image according to an embodiment of the present invention. FIG. 2 illustrates receiving ultrasound echo signals at a plurality of angles according to an embodiment of the present invention.

A method for indicating medical equipment on an ultrasound image according to an embodiment of the present invention may include operation 110 of transmitting ultrasound signals to a target object, operation 120 of receiving ultrasound echo signals at a plurality of angles from the target object and equipment inserted in the target object, and operation 130 for obtaining location information of the equipment based on the ultrasound echo signals received at the plurality of angles.

Also, the method may include operation 140 of generating an image of the equipment based on the obtained location information, operation 150 of generating a basic image based on the ultrasound echo signals received at a predetermined angle, operation 160 for synthesizing the image of the equipment and the basic image, and operation 170 for displaying the synthesized image.

According to an embodiment of the present invention, in operation 110, the ultrasound signals transmitted toward the target object may include focused waves or plane waves.

Operation 120 of receiving the ultrasound echo signals at the plurality of angles may further include an operation of storing each of the ultrasound echo signals according to each of the plurality of angles at which the ultrasound echo signals are received. Referring to FIG. 2 in this regard, ultrasound signals transmitted toward a target object 200 and an equipment 201 inserted in the target object 200 may be received at a plurality of angles, for example, θ1, θ2 through θn. For example, receiving angles at which the ultrasound echo signals are received according to an embodiment of the present invention may include angles between ultrasound scan lines (N1, N2 through Nm) and the ultrasound echo signals themselves. That is, the ultrasound echo signals are each stored according to each of the plurality of angles at which the ultrasound echo signals are received.

Also, operation 120 of receiving the ultrasound echo signals at the plurality of angles may further include an operation of beamforming an ultrasound echo signal which is received at any one of the plurality of angles. For example, the ultrasound echo signals corresponding to the ultrasound signals transmitted toward the target object and the equipment inserted in the target object may be sorted according to each of the receiving angles and be focused. Also, as described above, the operation of beamforming according to an embodiment of the present invention may include sorting and focusing the ultrasound echo signals according to the receiving angles at which the ultrasound echo signals are received from the target object and the equipment inserted in the target object. Also, the operation of beamforming of the received ultrasound echo signals may be referred to as "reception beamforming".

Operation 130 of obtaining the location information of the equipment based on the received ultrasound echo signals at the plurality of angles according to an embodiment of the present invention may include an operation of determining an inserting angle of the equipment based on a receiving angle at which a received ultrasound echo signal has maximum amplitude (hereinafter, referred to as "receiving angle having maximum amplitude"). According to an embodiment of the present invention, the location information of the equipment may include information of the inserting angle of the equipment.

The receiving angle having maximum amplitude according to an embodiment of the present invention may include an angle formed by the receiving angle and the inserting angle of the equipment which may be any one angle of 80 degrees to 100 degrees. That is, the receiving angle having maximum amplitude may be obtained within a range of deflected angles of about 80 degrees to about 100 degrees from the inserting angle of the equipment.

Preferably, the receiving angle having maximum amplitude may include the receiving angle forming 90 degrees with the inserting angle of the equipment. For example, as shown in FIG. 2, the ultrasound echo signal received at an angle, θ_{K}, among the plurality of angles at which the ultrasound echo signals are received forms a right angle, i.e., 90 degrees, with the inserting angle of the equipment, and thus the inserting angle of the equipment may be determined to be θ_{K} accordingly.

Generally, when the receiving angle of the ultrasound echo signal and the inserting angle of the equipment form a right angle, the amplitude of the ultrasound echo signal may have the maximum height. Also, when the receiving angle of the ultrasound echo signal and the inserting angle of the equipment form a right angle, the equipment inserted in the target object may be visualized relatively clearly compared to at the other receiving angles.

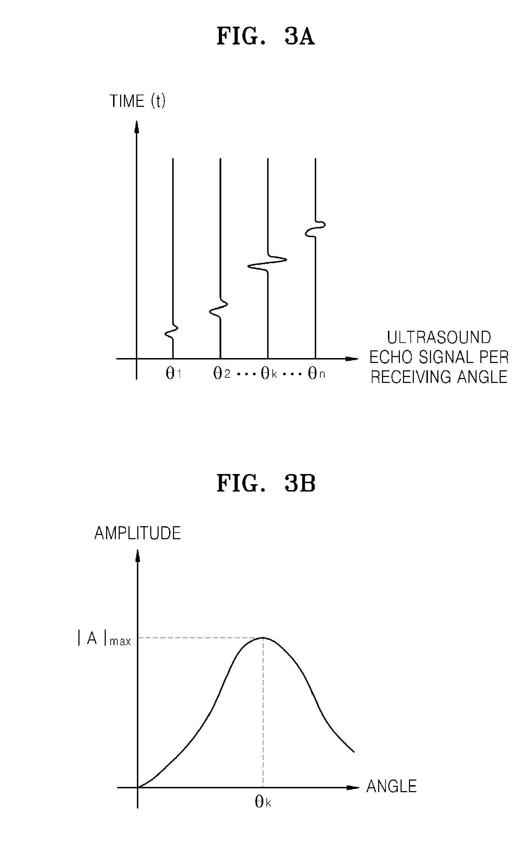

FIG. 3A illustrates ultrasound echo signals according to a plurality of angles at which the ultrasound echo signals are received according to an embodiment of the present invention. FIG. 3B illustrates a height of amplitude of an ultrasound echo signal per each of receiving angles at which the ultrasound echo signals are received. As shown in FIG. 3A, an amplitude of the ultrasound echo signal received at an angle of θ_{K} among the plurality of angles at which the ultrasound echo signals are received is relatively higher than amplitudes of ultrasound echo signals received at the other receiving angles. Referring to FIG. 3B, which illustrates an amplitude of an ultrasound echo signal per each angle at which the ultrasound echo signal is received, the ultrasound echo signal may have maximum amplitude at the receiving angle of θ_{K}. Also, θ_{K} may be 90 degrees and may be formed by the receiving angle of the ultrasound echo signal and the inserting angle of the equipment.

Operation 130 of obtaining the location information of the equipment based on the received ultrasound echo signals at the plurality of angles according to an embodiment of the present invention may further include an operation of obtaining a plurality of 2D images based on the ultrasound echo signal at the receiving angle having maximum amplitude and an operation of obtaining the location information of the equipment based on image information of the obtained plurality of 2D images. The image information according to an embodiment of the present invention may include brightness information of the plurality of 2D images, and the location information of the equipment may further include location information of a terminal end of the equipment.

According to an embodiment of the present invention, the plurality of 2D images of the target object and the equipment may be obtained based on the ultrasound echo signal at the receiving angle having maximum amplitude, for example at θ_{K}, and the location information of the terminal end of the equipment may be obtained based on the brightness information of the obtained plurality of 2D images. Preferably, the operation of obtaining the location information of the equipment based on the image information of the obtained plurality of 2D images may include an operation of obtaining the location information of the terminal end of the equipment based on the different brightness information in between the plurality of 2D images obtained from the ultrasound echo signal received at the receiving angle having maximum amplitude.

The brightness information according to an embodiment of the present invention may be variable depending on movement of tissues of the target object when the equipment is inserted into the target object, wherein the movement is due to insertion of the equipment into the target object. In other words, the difference in the brightness information may include a difference between brightness of an image region of tissues with increased mobility of the target object due to insertion of the equipment and brightness of an image region of the other tissues by sequentially accumulating the obtained plurality of 2D images. The location of the terminal end of the equipment inserted in the target object may be indicated using the difference in the brightness information.

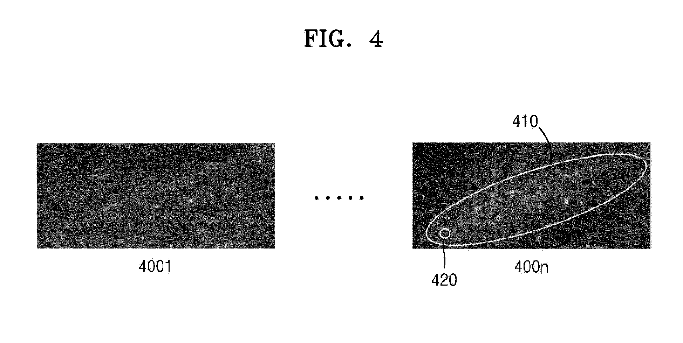

FIG. 4 illustrates obtaining of location information of equipment using image information of a plurality of 2D images, which are obtained based on an ultrasound echo signal according to an embodiment of the present invention.

According to an embodiment of the present invention, an image region 410 of tissues of which mobility is generated due to insertion of the equipment may be detected by a difference in brightness information of a plurality of 2D images 4001 through 400n. Also, a region 420 where a terminal end of the equipment is located may be detected based on the difference in the brightness of the plurality of 2D images 4001 through 400n. As described above, the location information of the equipment according to an embodiment of the present invention may further include the location information of the terminal end of the equipment as well as the information of the inserting angle of the equipment. According to an embodiment of the present invention, the terminal end location of the equipment may be determined at a region of the tissues where a brightness change rate is the maximum. In other words, a brightness difference of before and after the insertion of the equipment occurs between the tissues of which mobility is generated and the other tissues due to the insertion of the equipment, and thus the terminal end of the equipment may be detected based on the brightness difference. For example, a region where the brightness difference of before and after the insertion of the equipment is maximum may be detected as the terminal end location.

Operation 140 of generating the image of the equipment based on the obtained location information of the equipment according to an embodiment of the present invention may include segmenting an equipment region from the plurality of 2D images based on the location information of the equipment and controlling clarity of the equipment region. The image of the equipment according to an embodiment of the present invention may include an image of which clarity of the equipment region is controlled. In other words, the image of the equipment may include an image of the equipment region with improved clarity by segmenting the equipment region from a tissue region of the target object. For example, clarity of the equipment region may be improved by segmenting the tissues of the target object and the equipment by using the difference in the brightness information between the plurality of 2D images and controlling the brightness of the equipment region of at least one image from among the plurality of 2D images. The equipment region may be segmented using a Hough transform-based needle segmentation algorithm, etc.

Operation 150 of generating the basic images based on the ultrasound echo signal which is received at a predetermined angle according to an embodiment of the present invention may include generating an image of the target object by using ultrasound signals which are transmitted to and received from the target object at a pre-set angle, and the generated image may be the basic image. For example, the basic image according to an embodiment of the present invention may include a brightness mode (B mode) image. Also, according to an embodiment of the present invention, the predetermined angle may include the angle at which the ultrasound echo signal has maximum amplitude.

Operation 160 of synthesizing the image of the equipment and the basic image according to an embodiment of the present invention may be implemented by overlaying the image of the equipment on the basic image. For example, the image of the equipment and the basic image may be synthesized by using a cutout process, which cuts out a certain portion of an image, a warping process, which changes size, location, or a rotation angle of the cutout image through 3D image conversion, or an alpha blending method, which mixes a plurality of images with an appropriate ratio.

A method of indicating medical equipment on an ultrasound image according to an embodiment of the present invention may further include operation 170 of displaying the synthesized image.

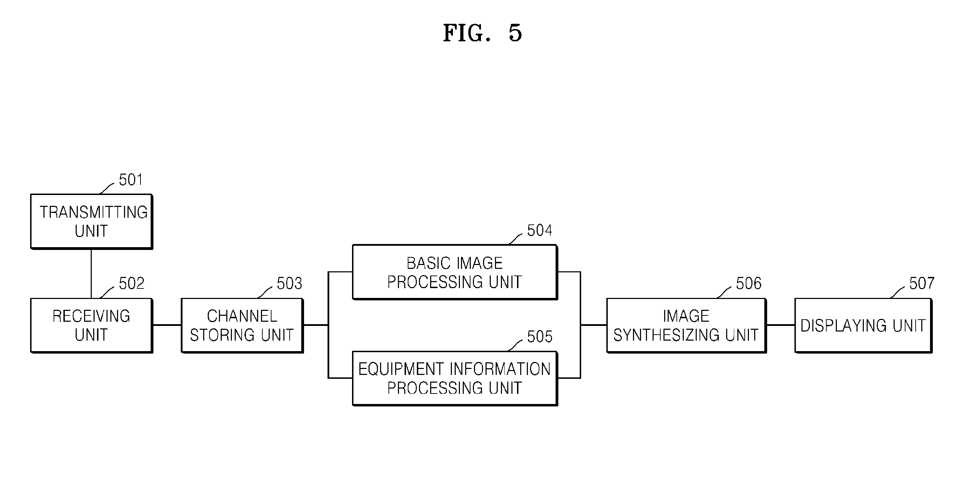

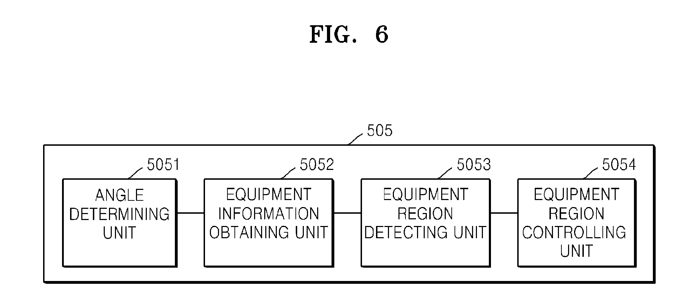

FIG. 5 illustrates an apparatus for indicating medical equipment on an ultrasound image according to an embodiment of the present invention. FIG. 6 illustrates an equipment information processing unit according to an embodiment of the present invention.

The apparatus for indicating a medical equipment on an ultrasound image according to an embodiment of the present invention may include a transmitting unit 501 for transmitting ultrasound signals toward a target object, a receiving unit 502 for receiving ultrasound echo signals at a plurality of angles from the target object and equipment that is inserted in the target object, and an equipment information processing unit 505 for obtaining location information of the equipment based on the ultrasound echo signals received at the plurality of angles and generating an image of the equipment based on the obtained location information of the equipment.

Also, the apparatus for indicating medical equipment on an ultrasound image according to an embodiment of the present invention may include a basic image processing unit 504 for generating the basic image based on the ultrasound echo signal received at a predetermined angle and an image synthesizing unit 506 for synthesizing the image of the equipment and the basic image. Moreover, the apparatus for indicating medical equipment on an ultrasound image according to an embodiment of the present invention may further include a displaying unit 507 for displaying the synthesized image.

The ultrasound signals transmitted toward the target object according to an embodiment of the present invention may include focused waves or plane waves.

According to an embodiment of the present invention, the apparatus for indicating medical equipment on an ultrasound image may further include a channel storing unit 503 for storing the ultrasound echo signals received at a plurality of angles by the receiving unit 502 according to each angle of the plurality of angles.

The equipment information processing unit 505 according to an embodiment of the present invention may include an angle determining unit 5051 for determining an inserting angle of the equipment based on a receiving angle at which a received ultrasound echo signal has maximum amplitude (hereinafter, referred to as "receiving angle having maximum amplitude").

The location information of the equipment may include information of the inserting angle of the equipment. The receiving angle having maximum amplitude may be 90 degrees and may be formed by the receiving angle and the inserting angle of the equipment. For example, as described above, the receiving angle having maximum amplitude may correspond to the inserting angle of the equipment. In other words, when the receiving angle of the ultrasound echo signal and the inserting angle of the equipment form a right angle, the amplitude of the ultrasound echo signal may have the maximum height. Also, in this case, the equipment may be visualized relatively clearly compared to at the other receiving angles.

Referring to FIGS. 3A and 3B, amplitude of the ultrasound echo signal received at an angle of θ_{K} among the plurality of angles at which the ultrasound echo signals are received is relatively higher than amplitudes of the ultrasound echo signals received at any other receiving angles. Here, the receiving angle θ_{K} may be determined as the inserting angle. As described above, generally when the receiving angle of the ultrasound echo signal and the inserting angle of the equipment form a right angle, the amplitude of the ultrasound echo signal may have the maximum height.

The equipment information processing unit 505 according to an embodiment of the present invention may further include an equipment information obtaining unit 5052 for obtaining a plurality of 2D images based on the ultrasound echo signal at the receiving angle having maximum amplitude and obtaining location information of the equipment based on image information of the obtained plurality of 2D images. The image information according to an embodiment of the present invention may include brightness information of the plurality of 2D images, and the location information of the equipment may further include location information of the terminal end of the equipment.

According to an embodiment of the present invention, the equipment information obtaining unit 5052 may obtain a plurality of 2D images of the target object and the equipment based on the ultrasound echo signal at the receiving angle having maximum amplitude, for example at θ_{K}, and obtain the location information of the terminal end of the equipment based on the brightness information of the obtained plurality of 2D images. Preferably, the equipment information obtaining unit 5052 may obtain the location information of the terminal end of the equipment based on the different brightness information in between the plurality of 2D images obtained from the ultrasound echo signal received at the receiving angle having maximum amplitude.

The brightness information according to an embodiment of the present invention may be variable depending on movement of tissues of the target object when the equipment is inserted into the target object, wherein the movement is due to insertion of the equipment into the target object. In other words, the difference in the brightness information may include a difference between brightness of an image region of tissues with increased mobility of the target object due to insertion of the equipment and brightness of an image region of the other tissues by sequentially accumulating the obtained plurality of 2D images. The location of the terminal end of the equipment inserted in the target object may be indicated using the difference in the brightness information.

Referring back to FIG. 4, the equipment information obtaining unit 5052 according to an embodiment of the present invention may detect an image region 410 of tissues of which mobility is generated due to insertion of the equipment by the difference in brightness information of a plurality of 2D images 4001 through 400n. Also, a region 420 where a terminal end of the equipment is located may be detected based on the difference in the brightness of the plurality of 2D images 4001 through 400n. As described above, the location information of the equipment according to an embodiment of the present invention may further include the location information of the terminal end of the equipment as well as the information of the inserting angle of the equipment.

The equipment information processing unit 505 may further include an equipment region detecting unit 5053 for segmenting an equipment region from the plurality of 2D images based on the location information of the equipment and an equipment region controlling unit 5054 for controlling clarity of the equipment region.

The image of the equipment according to an embodiment of the present invention may include an image of which clarity of the equipment region is controlled. In other words, the image of the equipment may include an image of the equipment region with improved clarity by segmenting the equipment region from a tissue region of the target object. For example, clarity of the equipment region may be improved by segmenting the tissues of the target object and the equipment by using the difference in the brightness information between the plurality of 2D images and controlling the brightness of the equipment region of at least one image among the plurality of 2D images. The equipment region may be segmented using a Hough transform-based needle segmentation algorithm, etc.

The basic image processing unit 504 according to an embodiment of the present invention is for generating an image of the target object by using ultrasound signals which are transmitted to and received from the target object at a predetermined angle, and the generated image may be the basic image. For example, the basic image according to an embodiment of the present invention may include a brightness mode (B mode) image. Also, according to an embodiment of the present invention, the predetermined angle may include the angle at which the ultrasound echo signal has maximum amplitude.

The image synthesizing unit 506 according to an embodiment of the present invention is for implementing the image of the equipment and the basic image by overlaying the image of the equipment on the basic image. As described above, the image of the equipment and the basic image may be overlaid by using a cutout process, a warping process, or an alpha blending method.

The embodiments of the present invention may be written as computer programs and may be implemented in general-purpose digital computers that execute the programs using a computer readable recording medium. Examples of the computer readable recording medium include magnetic storage media (e.g., ROM, floppy disks, hard disks, etc.), optical recording media (e.g., CD-ROMs, or DVDs), etc.

While the present invention has been particularly shown and described with reference to exemplary embodiments thereof, it will be understood by those of ordinary skill in the art that various changes in form and details may be made therein without departing from the spirit and scope of the present invention as defined by the following claims. The preferred embodiments should be considered in a descriptive sense only and not for purposes of limitation. Therefore, the scope of the invention is defined not by the detailed description of the invention but by the appended claims, and all differences within the scope will be construed as being included in the present invention.

## Claims

1. A method of indicating medical equipment on an ultrasound image, the method comprising:
transmitting ultrasound signals toward a target object;
receiving ultrasound echo signals at a plurality of angles from the target object and equipment inserted in the target object;
obtaining location information of the equipment based on the ultrasound echo signals received at the plurality of angles;
generating an image of the equipment based on the obtained location information;
generating a basic image based on the ultrasound echo signals received at a predetermined angle; and
synthesizing the image of the equipment and the basic image.

2. The method of claim 1, wherein the ultrasound signals transmitted toward the target object comprise focused waves or plane waves.

3. The method of claim 1, wherein the receiving of the ultrasound echo signals at the plurality of angles further comprises storing each of the ultrasound echo signals according to each of the plurality of angles at which the ultrasound echo signals are received.

4. The method of claim 3, wherein the obtaining of the location information of the equipment based on the received ultrasound echo signals at the plurality of angles comprises determining an inserting angle of the equipment based on a receiving angle at which a received ultrasound echo signal has maximum amplitude, wherein the location information of the equipment comprises information of the inserting angle of the equipment.

5. The method of claim 4, wherein the receiving angle with respect to the maximum amplitude comprises an angle formed by the receiving angle and the inserting angle of the equipment which is an angle in a range of 80 degrees to 100 degrees.

6. The method of claim 4, wherein the obtaining of the location information of the equipment based on the received ultrasound echo signals at the plurality of angles further comprises:
obtaining a plurality of 2-dimensional (2D) images based on the ultrasound echo signal at the receiving angle with respect to the maximum amplitude; and
obtaining the location information of the equipment based on image information of the obtained plurality of 2D images,
wherein the image information comprises brightness information of the plurality of 2D images, and the location information of the equipment further comprises location information of a terminal end of the equipment.

7. The method of claim 6, wherein the obtaining of the location information of the equipment based on the image information of the obtained plurality of 2D images comprises obtaining the location information of the terminal end of the equipment based on the different brightness information in between the plurality of 2D images obtained from the ultrasound echo signal received at the receiving angle having maximum amplitude, wherein the brightness information is variable depending on movement of tissues of the target object when the equipment is inserted into the target object, wherein the movement is due to insertion of the equipment into the target object.

8. The method of claim 7, wherein the generating of the image of the equipment based on the obtained location information of the equipment comprises:
segmenting an equipment region from the plurality of 2D images based on the location information of the equipment; and
controlling clarity of the equipment region,
wherein the image of the equipment comprises an image in which clarity of the equipment region is controlled.

9. The method of claim 8, wherein the generating of the basic image based on the ultrasound echo signal which is received at a predetermined angle comprises generating an image of the target object by using ultrasound signals which are transmitted to and received from the target object at a pre-set angle, wherein the generated image is the basic image.

10. The method of claim 1, wherein the method further comprises displaying the synthesized image.

11. The method of claim 1, wherein the receiving of the ultrasound echo signals at the plurality of angles further comprises beamforming an ultrasound echo signal which is received at any one of the plurality of angles.

12. An apparatus for indicating medical equipment on an ultrasound image, the apparatus comprising:
a transmitting unit for transmitting ultrasound signals toward a target object;
a receiving unit for receiving ultrasound echo signals at a plurality of angles from the target object and equipment that is inserted in the target object;
an equipment information processing unit for obtaining location information of the equipment based on the ultrasound echo signals received at the plurality of angles and generating an image of the equipment based on the obtained location information of the equipment;
a basic image processing unit for generating a basic image based on the ultrasound echo signal received at a predetermined angle; and
an image synthesizing unit for synthesizing the image of the equipment and the basic image.

13. The apparatus of claim 12, wherein the apparatus further comprises a channel storing unit for storing the ultrasound echo signals received at a plurality of angles by the receiving unit according to each angle of the plurality of angles.

14. The apparatus of claim 12, wherein the apparatus comprises a displaying unit for displaying the synthesized image.

15. A computer-readable recording medium storing a computer-readable program for executing the method of claim 1.
